# EUROPEAN PATENT APPLICATION

(11) **EP 1 543 842 A1**
(43) Date of publication of application: **22.06.2005**
(21) Application number: 04029451.4
(22) Date of filing: 13.12.2004
(51) Int. Cl.: A61L 2/00

(54) **Method of sterilizing a biocompatible material**

(30) Priority: 15.12.2003 JP 2003417039
(71) Applicant: Nipro Corporation, Kita-ku, Osaka 531-8510 (JP)
(72) Inventor: Matsuda, Kazuhisa Nipro Corporation, Osaka-shi Osaka, 531-8510 (JP); Morinaga, Yukihiro Nipro Corporation, Osaka-shi Osaka, 531-8510 (JP); Kamimura, Ryosuke Nipro Corporation, Osaka-shi Osaka, 531-8510 (JP)
(74) Representative: Albrecht, Thomas, Dr.

(57) **Abstract**

A method of sterilizing a biocompatible material with which decomposition and deterioration in a process of radiation sterilization can be suppressed by hermetically wrapping the biocompatible material together with a deoxidizer with a nonbreathable wrapping material and subjecting the resultant to radiation sterilization. The method suppresses decomposition and deterioration of the material in a process of radiation sterilization. More particularly, the method can reduce the effects of decomposition and deterioration on decomposition time of the biocompatible material in living organisms.

## Description

### Field of the Invention

The present invention relates to a method of sterilizing a biocompatible material with which decomposition and deterioration in a process of radiation sterilization can be suppressed.

### Background of the invention

In recent years, there has been considerable development in the field of regenerative medicine aimed at regeneration of functions of tissues and organs of a living organism that have suffered functional disorder or dysfunction by positively utilizing cells. Technologies that regenerate various organs such as gingivae, bones, blood vessels, nerves, and serous membranes have been established. To regenerate those organs, defective parts of the organs are supplemented by using supplementing instruments that are made of biocompatible materials and enable induction and regeneration of tissues to perform regenerative induction of the organs. At the same time, the biocompatible materials are decomposed or absorbed in the living organism. Further, attempts have been made in order to control time of decomposition/absorption of the biocompatible materials in the living organisms and increase the supplementing strength by crosslinking the biocompatible materials.

On the other hand, since the biocompatible materials are medical materials, a sterilization process is indispensable. Sterilization methods that are currently applied to medical materials include high-pressure vapor sterilization, ethylene oxide gas (EOG) sterilization, and radiation sterilization. The high-pressure vapor sterilization may be used for any medical material as far as it can endure high temperatures and high pressures. However, few biocompatible materials can endure high temperatures and high pressures. The EOG sterilization is excellent in that it suppresses the deterioration of the material. However, there is the possibility that residual EOG has an effect on living organisms. The radiation sterilization has a higher capability of sterilization than the high-pressure vapor sterilization and the ethylene oxide gas sterilization and is one of the most noteworthy methods. The radiation includes α-rays, β-rays, γ-rays, neutron beams, electron beams, and X-rays. However, the radiation sterilization using such radiation may cause decomposition or deterioration and have severe influences on physical properties of the material. In particular, in the case of biocompatible materials, they cause a change in decomposition time. It is believed that such decomposition and deterioration are caused by a reaction of free radicals generated concomitantly with the irradiation with the radiation of oxygen. Those problems have not been solved by mere deaeration-wrapping of the biocompatible material with a nonbreathable material.

Disclosed as a biocompatible material that has solved the above-mentioned problem and enabled radiation sterilization is an invention that relates to a bone regeneration material made of a mixture of collagen and a mineral which is subjected to γ-ray sterilization to achieve a high sterilization insurance level (JP 63-132664 A). However, it does not mean that other biocompatible materials that contain no mineral can also be sterilized. Further, a method is disclosed for sterilization of collagen gel that contains a radiation protective material and water (JP 11-137662 A and JP 2000-107278 A). However, those biocompatible materials are applied only to water-swelled gels. Further, a method in which generation of high molecular weight radicals is suppressed by addition of a polyfunctional triazine-based compound is disclosed (JP 2003-695 A). However, the influence of the triazine compound on living organisms is unknown. Therefore, for biocompatible materials other than the above-mentioned, in particular, those biocompatible materials that are not gels and indispensably need to be stored in a dry state, the problems caused by radiation sterilization have not been solved.

### Summary of the Invention

Accordingly, a method of sterilizing a biocompatible material with which decomposition and deterioration can be suppressed in a process of radiation sterilization has been demanded.

The present invention relates to a method of sterilizing a biocompatible material with which decomposition and deterioration can be suppressed in a process of radiation sterilization.

That is, the present invention relates to:
(1) a method of sterilizing a biocompatible material, characterized by hermetically wrapping the biocompatible material together with a deoxidizer with a nonbreathable wrapping material and subjecting the resultant to radiation sterilization;
(2) the method of sterilizing a biocompatible material according to (1), in which the radiation sterilization is eithery-ray sterilization or electron beam sterilization;
(3) the method of sterilizing a biocompatible material according to (1), in which the biocompatible material has a shape selected from a sheet, fiber, woven fabric, non-woven fabric, porous body, tube, or combinations of two or more of these, and is molded from a biopolymer substance and/or a biodegradable polymer substance;
(4) the method of sterilizing a biocompatible material according to (3), in which the biopolymer substance is polysaccharide, DNA, polypeptide, or collagen;
(5) the method of sterilizing a biocompatible material according to (3), in which the biodegradable polymer substance is polyamide, polyester, polylactic acid, or polyglycol acid;
(6) the method of sterilizing a biocompatible material according to (1), characterized in that a biocompatible material molded from collagen and/or hyaluronic acid is wrapped together with a deoxidizer with a nonbreathable wrapping material and the resultant is subjected to radiation-sterilization; and
(7) the method of sterilizing a biocompatible material according to (1), characterized by hermetically wrapping the biocompatible material together with a deoxidizer and a desiccant with a nonbreathable wrapping material and subjecting the resultant to radiation sterilization.

The method of sterilizing a biocompatible material according to the present invention suppresses decomposition and deterioration of the material in a process of radiation sterilization. More particularly, the method can reduce the effects of decomposition and deterioration on decomposition time of the biocompatible material in living organisms.

### Detailed Description

The method of sterilizing a biocompatible material according to the present invention is characterized by radiation sterilization. In the radiation sterilization, the radiation includes α-rays, β-rays, γ-rays, neutron beams, electron beams, and X-rays. γ-Ray sterilization and electron beam sterilization are preferable. The sterilization methods may be ordinary methods that can be performed by one skilled in the art. The dose of radiation is 10 to 50 kGy, preferably 20 to 30 kGy. The temperature condition is 15 to 35°C, preferably 20 to 30°C.

The biocompatible material used in the present invention is characterized in that it is hermetically wrapped together with a deoxidizer with a nonbreathable wrapping material. In the hermetic wrapping, air may remain in the wrapped body. However, the wrapping is preferably performed in a deaerated state or an inert gas-filled state. The form of the wrapping material can be vessel-like or bag-like, however, the bag-like form which is advantageous in view of maintenance and cost is preferable.

The biocompatible material used in the present invention is a material that is prepared for the purpose of supplementing or inducing and regenerating damaged organs or tissues in living organisms and that is decomposed/absorbed or remains in the living organisms without having influences on the living organisms when embedded therein.

The shape of the biocompatible material is not particularly limited but may be a sheet, fiber, woven fabric, non-woven fabric, porous body, or tube, or a combination of two or more of these. These biocompatible materials are preferably subjected to dehydration crosslinking treatment. The methods of dehydration crosslinking include a crosslinking method with heat and a crosslinking method with a crosslinking agent (for example, glutaraldehyde). Of these, the crosslinking with heat is preferable. Advantageously, the degree of crosslinking treatment can control the decomposition time of the material in living organisms.

Further, the biocompatible materials include: bioabsorbable polymer substances such as collagen, hyaluronic acid, and chitin; and biodegradable polymer substances such as polyester, polyamide, and polylactic acids. Bioabsorbable polymer substances are preferable, and collagen, hyaluronic acid, and so on that have functional groups capable of being subjected to crosslinking treatment are more preferable.

Examples of such biocompatible materials include: a membrane-like product made of collagen non-woven fabric having collagen sponge laminated on both sides thereof as disclosed in JP 2000-69961 A; a porous substance made from lactic acid and caprolactam as disclosed in JP 2000-197693 A; a medical film made of polylactic acid and aliphatic polyester as disclosed in JP 2000-189509 A; and a collagen tube having collagen sponge and collagen fiber filled in the cavity thereof as disclosed in JP 2002-320630 A.

The deoxidizer in the present invention generally means a substance that has a capability of taking away oxygen from a counterpart, that is, a biocompatible material, regardless of the amount or effect thereof. However, the stronger the effect, the more preferable. In order that the deoxidizer may be hermetically wrapped together with the biocompatible material to be embedded in a living organism, the deoxidizer must be nontoxic, must generate no other gas when it absorbs oxygen, and must generate no other gas or not be inactivated when it is irradiated with radiation. Examples of such deoxidizers include iron, zinc, copper, and tin, and those composed mainly of active iron oxide are preferable. Examples of commercially available deoxidizers include Sansocut (trade name, manufactured by Nittetsu Fine Products Co., Ltd.), Ageless (trade name, manufactured by Mitsubishi Gas Chemical Corporation), Tamotsu (trade name, manufactured by Oji Duck Co., Ltd.), Wellpack (trade name, manufactured by Taisei Co., Ltd.), and A500-HS Oxygen Absorber (trade name, manufactured by I. S. O. Co., Ltd.). In addition thereto, sugars, polysaccharides, vitamin C, L-ascorbic acid, erythorbic acid, activated carbon, chitin-based activated carbon, chitosan-based activated carbon, cellulose-based activated carbon, zeolite, carbon molecular sieve, silica gel, activated alumina, and so on may also be used.

The degree of removing oxygen with the above-mentioned deoxidizers is preferably such that the oxygen concentration at 25°C in an air atmosphere is about 1 mg/l or less. Values lower than this are preferable, however, the present invention is not limited to this.

Further, in sterilizing biocompatible materials that need to be stored in a dry state, it is preferable that the materials be wrapped together with a desiccant. Preferably, the desiccant is nontoxic, generates no other gas when it absorbs oxygen, and generates no other gas or is not inactivated when it is irradiated with radiation, as is the case with the deoxidizer. An examples of commercially available desiccants includes ID Sheet (trade name, manufactured by ID Co., Ltd.).

Further, the nonbreathable wrapping material in the present invention means a material that is difficult to be permeated with oxygen. Specifically, it is preferable that the material have an oxygen permeation coefficient at a temperature of 25°C and a humidity of 50% at atmospheric pressure of 5.0 × 10² cc/m²·hour/25 µm or less, and more preferably 1.0 × 10³ cc/m2·hour/25 µm or less. Suitable materials to be selected include polyester, polyvinylidene chloride, polyvinylidene chloride-coated polyester, polyvinyl chloride-coated polypropylene, polyvinyl alcohol, poly(ethylene/vinyl alcohol) copolymers, aluminum-deposited polyethylene, aluminum-deposited polyester, and silica-coated polyester.

Further, in addition to the above-mentioned nonbreathability, the nonbreathable material preferably is a material that is easy to be molded and processed, is durable to radiation sterilization, blocks light from the outside, and is difficult to permeate water vapor. Therefore, it can be said that a laminate sheet that includes polyethylene as an outer layer, aluminum as an intermediate layer, and polyethylene as an inner layer is the most suitable material.

### [Example]

Hereinafter, the present invention will be described by way of detailed examples. However, the present invention should not be considered to be limited to these examples.

### [Reference Example 1]

150 ml of a 7 wt% aqueous solution of acid-soluble collagen (manufactured by Nippon Ham Co., Ltd.; SOFD type, Lot No. 0102226) were extruded in 3 liters of 99.5 vol% ethanol (manufactured by Wako Pure Chemical Industry Co., Ltd., special grade) coagulation bath to dehydrate and coagulate the collagen. The obtained collagen fiber was laminated to form a collagen non-woven fabric. Then, the obtained collagen non-woven fabric was air-dried in a clean bench and subsequently subjected as it was to a heat dehydration crosslinking reaction in a vacuum dry oven (manufactured by EYELA corporation: VOS-300VD type) at 120°C for 24 hours under high vacuum (1 torr or less). After completion of the crosslinking reaction, to fill the interstices between the fibers of the crosslinked collagen non-woven fabric, a 1 wt% aqueous solution of collagen was coated into the collagen non-woven fabric as a binder treatment and the resultant was dried. Repeating each of the coating operation and the drying operation three times resulted in a non-woven fabric layer made of collagen fiber. After that, the layer was heated at 120°C for 12 hours in the vacuum dry oven under high vacuum (1 torr or less) to subject the coated collagen to a heat dehydration crosslinking reaction. After completion of the crosslinking reaction, the collagen membrane-like product was immersed in an aqueous solution of sodium hydrogen carbonate (7.5 wt%) for 30 minutes to perform a neutralization treatment and then taken out from the aqueous solution of sodium hydroxide. The residual sodium hydroxide on the surface of the non-woven fabric layer made of collagen fiber was washed with distilled water and the non-woven fabric was air-dried in the clean bench to obtain a collagen membrane-like product.

### [Example 1]

The collagen membrane-like product prepared in Reference Example 1 was wrapped together with a deoxidizer with an aluminum wrapping material made of polyethylene/aluminum-deposited film/polyethylene (the volume enclosed by the wrapping being 1,200 ml) in a state where the amount of air enclosed in the wrapping was about 500 ml. A500-HS Oxygen Absorber (trade name, manufactured by I. S. O. Co., Ltd.) was used as the deoxidizer. The wrapped collagen membrane-like product was subjected to Y-ray sterilization at about 20°C. On this occasion, the dose of Y-rays was 25 kGy.

### [Comparative Example 1]

Only the collagen membrane-like product prepared in Reference Example 1 was wrapped with the same aluminum wrapping material as that in Example (the volume enclosed by the wrapping being 1,200 ml) in a state where the amount of air enclosed in the wrapping was about 500 ml. The wrapped collagen membrane-like product was subjected to γ-ray sterilization at room temperature. On this occasion, the dose of γ-rays was 25 kGy.

### [Comparative Example 2]

Only the collagen membrane-like product prepared in Reference Example 1 was wrapped with the same aluminum wrapping material as that in Example (having a volume of 1,200 ml) in a state where the air in the wrapping was removed. The wrapped collagen membrane-like product was subjected to γ-ray sterilization at room temperature. On this occasion, the dose of γ-rays was 25 kGy.

### [Experiment 1]

The collagen membrane-like product of the Reference Example was measured for a single point supported tensile strength by using an autograph. Specifically, a 4-0 proline suture was crossed through the collagen membrane-like product at a position of 5 mm from an end thereof and a loop was formed to obtain a test piece. The loop-formed end was provided as an upper part of the test piece and the loop of 4-0 proline was engaged with a hook-like structure attached to the autograph. The part extending 10 mm from the lower end of the test piece was fixed with a chuck and measurement was made in this state. The measurement was performed 5 times. As a result, the unsterilized collagen membrane-like product had a single point supported tensile strength of 2.60 N.

The collagen membrane-like products of Example 1 and Comparative Examples 1 and 2 were taken out of the aluminum wrapping materials after γ-ray sterilization, and were measured for single point supported tensile strengths. Conditions of the measurements were the same as those described above.

Table 1 shows variances of single point supported tensile strengths obtained by subtracting each of the single point supported tensile strengths in Example 1 and Comparative Examples 1 and 2 as measured in Experiment 1 from the single point supported tensile strength [2.60 (N)] of the Reference Example (unsterilized). It is apparent that the single point supported tensile strength of the collagen membrane-like product in Example 1 showed a suppressed decrease in single point supported tensile strength as compared to the single point supported tensile strengths of the collagen membrane-like products in Comparative Examples 1 and 2 which had been sterilized without a deoxidizer.

**[Table 1]**

| | Example 1 | Comparative Example 1 | Comparative Example 2 |
|---|---|---|---|
| Variance of single point supported tensile strength (N) | 0.19 | 0.71 | 0.54 |

## Claims

1. A method of sterilizing a biocompatible material, **characterized by** comprising hermetically wrapping the biocompatible material together with a deoxidizer with a nonbreathable wrapping material and subjecting the resultant to radiation sterilization.

2. The method of sterilizing a biocompatible material according to claim 1, wherein the radiation sterilization is either γ-ray sterilization or electron beam sterilization.

3. The method of sterilizing a biocompatible material according to claim 1, wherein the biocompatible material has a shape selected from a sheet, fiber, woven fabric, non-woven fabric, porous body, tube, or combinations of two or more of these, and is molded from a biopolymer substance and/or a biodegradable polymer substance.

4. The method of sterilizing a biocompatible material according to claim 3, wherein the biopolymer substance comprises polysaccharide, DNA, polypeptide, or collagen.

5. The method of sterilizing a biocompatible material according to claim 3, wherein the biodegradable polymer substance comprises polyamide, polyester, polylactic acid, or polyglycol acid.

6. The method of sterilizing a biocompatible material according to claim 1, **characterized in that** a biocompatible material molded from collagen and/or hyaluronic acid is wrapped together with a deoxidizer with a nonbreathable wrapping material and the resultant is subjected to radiation-sterilization.

7. The method of sterilizing a biocompatible material according to claim 1, **characterized by** hermetically wrapping the biocompatible material together with a deoxidizer and a desiccant with a nonbreathable wrapping material and subjecting the resultant to radiation sterilization.
